# EUROPEAN PATENT APPLICATION

(11) **EP 4 116 869 A1**
(43) Date of publication of application: **11.01.2023**
(21) Application number: 21184360.2
(22) Date of filing: 07.07.2021
(51) Int. Cl.: G06K 9/00, G06K 9/62, G06N 3/04, G06T 7/00

(54) **A METHOD AND AN APPARATUS FOR PREDICTING A FUTURE STATE OF A BIOLOGICAL SYSTEM, A SYSTEM AND A COMPUTER PROGRAM**

(71) Applicant: Leica Microsystems CMS GmbH, 35578 Wetzlar (DE)
(72) Inventor: SERRA LLETI, José Miguel, 35578 Wetzlar (DE); KAPPEL, Constantin, 69198 Schriesheim (DE)
(74) Representative: 2SPL Patentanwälte PartG mbB

(57) **Abstract**

An embodiment of a method 100 for predicting a future state of a biological system is provided. The method 100 comprises receiving 101 a microscope image depicting the biological system at an associated time and receiving 102 metadata corresponding to the microscope image. The method 100 further comprises extracting 103 features from the microscope image having information on a state of the biological system and using 104 the features and the metadata to predict the future state of the biological system.

## Description

### Field

The present disclosure relates to future states of biological systems. In particular, examples relate to a method for predicting a future state of a biological system, an apparatus for predicting a future state of a biological system, a system and a computer program.

### Background

Biological systems, such as cells, are usually analyzed by light microscopy or fluorescence microscopy to obtain a magnified image of the biological system. The microscope image of the biological system can provide information on a current state, e.g. with respect to viability, of the biological system. Over time, the state of the biological system can change or deteriorate for many reasons. In the specific case of cells, for example cell death can be biologically induced by creating certain phenotypes, adding drugs or setting wrong environmental conditions.

There are some approaches to analyze and conclude on the state of the biological system. For example, analysis of particular features based on DNA fragmentation, cell membrane protein flipping, cell detachment from basal membrane or pyknosis or shrinkage is applied. Other methods are based on artificial neural networks (NN) being trained for classifying images according to the current state of the biological system.

However, so far conclusion regarding the state of the biological system is performed in a post-mortem analysis. For example, the biological system needs to be stained for proper assessment. This usually affects the state of the biological system significantly such that further assessment of the biological system is not possible. Further, there is a lack in determining parameters having a negative impact on the state of the biological system during a time-series (or time lapse) experiment.

### Summary

Hence, there is a desire for an improved technique for predicting a future state of a biological system.

This desire is addressed by the subject matter of the independent claims.

Some embodiments relate to a method for predicting a future state of a biological system. The method comprises receiving a microscope image depicting the biological system at an associated time and receiving metadata corresponding to the microscope image. The method further comprises extracting features from the microscope image having information on a state of the biological system and using the features and the metadata to predict the future state of the biological system. The microscope image may depict the biological system with higher resolution and may comprise information on the state of the biological system at that time the microscope image was generated by a microscope. Metadata, e.g. related to a surrounding of the biological system or a configuration of the microscope, corresponding to the same (associated) time can be provided. By feature extraction, the microscope image (and the information thereof) can be transformed into a set of features having an information on the state of the biological system. The extracted features and the received metadata can be used to predict the state of the biological system in the future. Since metadata is used, a reliable prediction regarding the future state can be achieved. Further, the state of the biological system might not be affected by the method for predicting the future state, e.g. since prediction may be enabled without staining or changing the state of the biological system. According to the proposed method, significant (e.g. risk) parameters contributing to the future state of the biological system can be determined.

According to some embodiments, the state of the biological system is related to at least one of a health, an activity, and a growth of the biological system. Health, activity and growth may be a good indicator for assessing the state of the biological system with respect to viability.

According to some embodiments, the metadata comprises information on at least one of a configuration of a microscope, used for generating the microscope image, a surrounding, an agent interacting with the biological system at the associated time, a temperature, a pH, a partial pressure of carbon dioxide, a partial pressure of oxygen, a humidity, a culture condition of the biological system, a type or amount of a buffer solution, nutrient, antibiotic or growth factor of the biological system at the associated time. Knowledge of one or more of the named parameters may be essential to be able to predict the future state of the biological system with good performance since the named parameters may be able to affect the state of the biological system significantly.

According to some embodiments, extracting features from the microscope image comprises using an encoder of a trained artificial NN (neural network) having an encoder-decoder architecture. Provision of features can be enabled by only using the encoder of the artificial NN The encoder of the artificial NN can be used as a feature extractor. The encoder may enable a transformation of raw data (comprising information on a state of the biological system) of the microscope image to a set of features having information on the state of the biological system. Accordingly, the encoder may enable a dimensionality reduction (almost) without loss of information on the state of the biological system. The extracted features may be compatible with another algorithm or model for predicting the future state.

According to some embodiments, using the features and the metadata comprises detecting anomalies by means of a further trained artificial NN, the further trained artificial NN being trained based on a sequence of microscope images, depicting biological systems over time, and a corresponding sequence of metadata over the time. Using a single microscope image with corresponding metadata may be sufficient for predicting the future state of the biological system by means of the further artificial NN. The further artificial NN may have learned to reliably identify anomalies in the microscope image due to the training based on the sequence of microscope images and corresponding sequence of metadata (both comprising information on different time steps).

According to some embodiments, the method further comprises receiving a further microscope image depicting the biological system at another associated time and receiving further metadata corresponding to the further microscope image. The method further comprises extracting further features from the further microscope image having information on the state of the biological system. Further, the method comprises using the features and the further features and the metadata and the further metadata to predict the future state by detecting anomalies based on a temporal development between the features and the further features and between the metadata and further metadata. Two or more microscope images with corresponding metadata being related to different time stamps can be used to predict the future state of the biological system. Since at least two microscope images with corresponding metadata are used, anomaly detection can be based on a trend over time related to the at least two microscope images and corresponding metadata for the specific biological object to be analyzed. Anomaly detection based on at least two microscope images and corresponding metadata can be achieved with less complexity, e.g. without the need for (training) a further artificial NN

According to some embodiments, the method further comprises using a decoder of the trained artificial neural network having the encoder-decoder architecture to reconstruct a segmented image based on the future state being predicted. The segmented image depicts the biological system as one or more segments according to the future state. The decoder of the trained artificial NN may reverse the process of encoding (for extracting the features of the biological system) yet based on the future state being predicted. In this way, a visual representation of the predicted future state of the biological object can be provided by reconstructing the segmented image.

According to some embodiments, the method further comprises identifying a risk parameter of the metadata. The risk parameter has a positive correlation with a degradation of the state of the biological system with respect to the future state. This means, the risk parameter may contribute to the degradation of state of the biological system in view of the time period between the associated time (and optionally the other associated time) and the time related to the future state. Knowledge on the risk parameter may be useful for the further workflow of a time lapse experiment to assess the state of the biological system.

According to some embodiments, the method further comprises generating data for an external entity having an influence on the risk parameter. The data comprises a command for the external entity to adapt a configuration related to the risk parameter for mitigating the degradation. The data comprising the command may be used to warn a user observing the biological system such that degradation of the biological system can be mitigated by manipulating the risk parameter. The data comprising the command may be also useable to feedback information on the risk parameter to a system, e.g. a microscope or an incubator, such that the system can manipulate a component related to the risk parameter, e.g. light source or thermostat, to mitigate the degradation of the biological system.

According to some embodiments, the risk parameter relates to an illumination property, a temperature, a humidity, an oxygen level, a carbon dioxide level or an agent having an influence on the state of the biological system.

Some embodiments relate to an apparatus for predicting a future state of a biological system. The apparatus is configured to receive a microscope image depicting a biological system at an associated time and receive metadata corresponding to the microscope image. The apparatus is further configured to extract features from the microscope image having information on a state of the biological system and use the features and the metadata to predict the future state of the biological system. The microscope image may depict the biological system with higher resolution and may comprise information on the state of the biological system at that time the microscope image was generated by a microscope. Metadata, e.g. related to a surrounding of the biological system or a configuration of the microscope, corresponding to that (associated) time can be provided. By feature extraction, the microscope image (and the information thereof) can be transformed into a set of features having an information on the state of the biological system. The extracted features and the received metadata can be used to predict the state of the biological system in the future. Since metadata is used, a reliable prediction regarding the future state can be achieved. Further, the state of the biological system might not be affected by the apparatus for predicting the future state, e.g. since prediction may be enabled without staining or changing the state of the biological system. According to the proposed apparatus, significant (e.g. risk) parameters contributing to the future state of the biological system can be determined.

Some embodiments relate to a system comprising a microscope configured to generate a microscope image depicting a biological system at an associated time. The system further comprises an apparatus for predicting a future state of a biological system as proposed above. The apparatus is configured to receive the microscope image to predict a future state of the biological system. The proposed apparatus can be used in combination with a microscope. The microscope generates one or more microscope images of the biological object to be analyzed. The proposed apparatus receives the microscope image and corresponding metadata for predicting the future state of the biological system. The corresponding metadata may relate to a configuration of the microscope for generating the microscope image at the associated time. Optionally, the apparatus may further identify a risk parameter and may generate corresponding feedback data to be used by the microscope such that degradation of the biological system can be mitigated.

Some embodiments relate to a computer program with a program code for performing the method according to one of the techniques described above when the computer program is executed by a processor. The computer program provides a program code for the proposed method which can be implemented in a software of an arbitrary apparatus. For example, the arbitrary apparatus may be a microscope, an incubator, or an external device supplying the microscope image and/or metadata. The computer program may be provided by a server to evaluate external data.

### Brief description of the Figures

Some examples of apparatuses and/or methods will be described in the following by way of example only, and with reference to the accompanying figures, in which
Fig. 1 illustrates a flow chart of an embodiment of a method for predicting a future state of a biological system;
Fig. 2 illustrates another flow chart of an embodiment of a method for predicting a future state of a biological system;
Fig. 3 illustrates examples of an input and an output of the trained artificial NN having an encoder-decoder architecture;
Fig. 4 illustrates a flowchart for generating training data for a trained artificial NN, having an encoder-decoder architecture, according to an example;
Fig. 5 illustrates a flowchart for generating segmented images by means of a trained artificial NN, having an encoder-decoder architecture, according to an example;
Fig. 6 illustrates a flowchart for the step of using the features and the metadata to predict the future state of the biological system according to an example;
Fig. 7 illustrates a flowchart for the step of reconstructing the segmented image based on the future state according to an example;
Fig. 8 illustrates another flowchart of an embodiment of a method for predicting a future state of a biological system;
Fig. 9 illustrates an example of a well plate useable in combination with the method for predicting the future state of a biological system;
Fig. 10a illustrates an embodiment of an apparatus for predicting a future state of a biological system; and
Fig. 10b illustrates an embodiment of a system comprising an apparatus for predicting a future state of a biological system; and
Fig. 11 illustrates an embodiment of a microscope system.

### Detailed Description

Some examples are now described in more detail with reference to the enclosed figures. However, other possible examples are not limited to the features of these embodiments described in detail. Other examples may include modifications of the features as well as equivalents and alternatives to the features. Furthermore, the terminology used herein to describe certain examples should not be restrictive of further possible examples.

Throughout the description of the figures same or similar reference numerals refer to same or similar elements and/or features, which may be identical or implemented in a modified form while providing the same or a similar function. The thickness of lines, layers and/or areas in the figures may also be exaggerated for clarification.

When two elements A and B are combined using an "or", this is to be understood as disclosing all possible combinations, i.e. only A, only B as well as A and B, unless expressly defined otherwise in the individual case. As an alternative wording for the same combinations, "at least one of A and B" or "A and/or B" may be used. This applies equivalently to combinations of more than two elements.

If a singular form, such as "a", "an" and "the" is used and the use of only a single element is not defined as mandatory either explicitly or implicitly, further examples may also use several elements to implement the same function. If a function is described below as implemented using multiple elements, further examples may implement the same function using a single element or a single processing entity. It is further understood that the terms "include", "including", "comprise" and/or "comprising", when used, describe the presence of the specified features, integers, steps, operations, processes, elements, components and/or a group thereof, but do not exclude the presence or addition of one or more other features, integers, steps, operations, processes, elements, components and/or a group thereof.

Fig: 1 shows a flowchart of an embodiment of a method 100 for predicting a future state of a biological system. The method 100 comprises receiving 101 a microscope image depicting the biological system at an associated time and receiving 102 metadata corresponding to the microscope image. The method 100 further comprises extracting features from the microscope image having information on a state of the biological system and using 104 the features and the metadata to predict the future state of the biological system.

The proposed method 100 can be used to make a forecast regarding the state of the biological system in the future based on the microscope image and the metadata. The microscope image illustrates the biological system and comprises information on the (current) state of the biological system at that time, the microscope image was captured by a microscope. At that time (named associated time), metadata can be collected and may comprise any information on parameters, e.g. possibly having (solely or in combination with other parameters) an influence on the biological system. By feature extraction, a dimensionality reduction can be achieved. In other words, information on the state of the biological system being given by the microscope image can be transformed to a set of features (almost) without data loss. The set of features can be combined with the metadata to be further processed by an algorithm to predict the feature state of the biological system. The proposed method 100 may be useable for time resolved experiments such that the state of the biological system can be evaluated, investigated, monitored or manipulated during the experiment. The method 100 may allow an analysis of the biological system prior to a critical degradation (or e.g. critical change in the state) of the biological system (rather than a post-mortem assessment). Since extracted features from the microscope image are used in combination with the corresponding metadata, a reliable prediction regarding the future state of the biological system can be given. According to the method, a reliable prediction on the future state can be made based on or more microscope images and corresponding metadata.

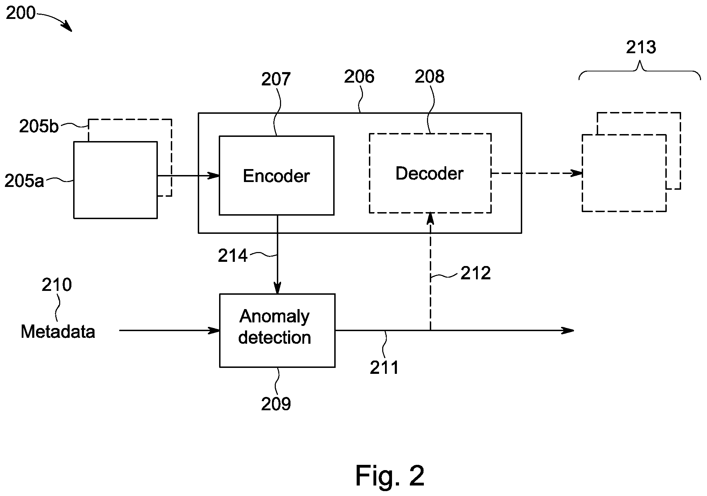

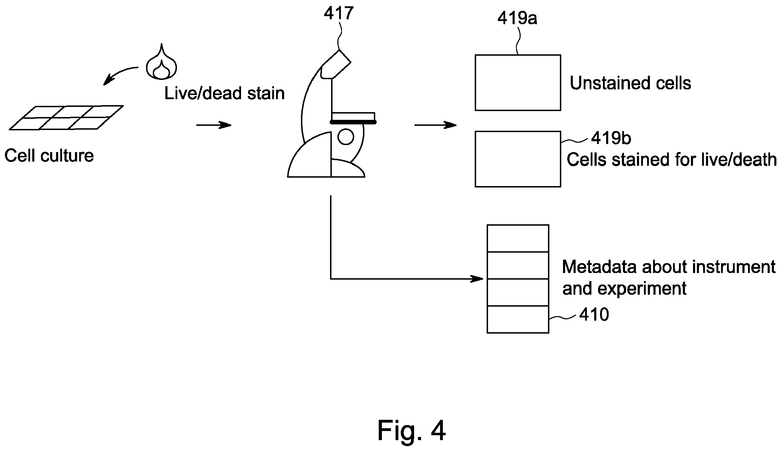

For example, feature extraction 103 can be enabled by using a trained artificial NN. The trained artificial NN may receive the microscope image as input data and may extract features from the microscope image having information on a state of the biological system. The trained artificial NN may comprise an encoder-decoder architecture and may only use the encoder (component) for extracting the features. In other words, the extracted features can be considered as intermediate data provided by the trained artificial NN. The extracted features may be a set of values, numbers (or symbols or suitable codes), a feature (state) vector, matrix or tensor. The extracted features may comprise at least one feature value related to the (current) state of the biological system. The extracted features, e.g. a feature vector, may comprise two or more feature (or state) values each representing the (current) state of the biological system in a similar or different manner. Further details on feature extraction are described in conjunction with Fig. 2 and Fig. 4.

The biological system may be any type of system being able to live, grow, divide, propagate, move, breed, develop, degrade and/or die. The biological system may have a metabolism, genome, exome, exposome, interactome, metabolome, lipidome, regulome, pharmacogenetics, transcriptome, secretome, epigenome, may have a set of biomolecules originating from one organism, may communicate with other biological systems and/or may be excitable by stimuli. For example, the biological system is one or more cells, organisms, such as protists, bacteria, archaea, plants, animals or fungi, or viruses in hosts.

The biological system can be characterized by its state. The state of the biological system can be described by the extracted features. For example, the state of the biological system is related to at least one of a health, an activity, and a growth of the biological system. The state of the biological system may be characterized by its color, size, shape, pattern, boundary, membrane, movement, (sexual) reproduction, division, growth, phenotype, general change in appearance, change due to stimuli such as drugs, active agents, chemicals, radiation, illumination, inflow of a substance of any aggregate phase, nutrients or the like. The state of the biological system may refer to the associated time when the microscope image was captured. Hence, the extracted features may describe the current state of the biological system.

At the associated time, metadata is provided. For example, the metadata comprises information on at least one of a configuration of a microscope (used for generating the microscope image), a surrounding, an agent interacting with the biological system at the associated time, a temperature, a pH, a partial pressure of carbon dioxide, a partial pressure of oxygen, a humidity, a culture condition of the biological system, a type or amount of a buffer solution, nutrient, antibiotic or growth factor of the biological system at the associated time. The metadata may comprise one or more static or dynamic parameters or information at the associated time the microscope image was captured.

For example, metadata may be any value coming from the microscope and/or any separate measuring device (e.g. any sensor output value) and may be stored together with the image acquired. If suitable, corresponding metadata may be stored independently from the microscope image. Metadata may reflect the current state of the microscope and/or the sample at the moment the image is saved. Metadata can be static (or common) which means that metadata can remain the same if more than one microscope image is acquired and used for future state prediction. Static metadata may refer to a resolution, dimensions, wavelengths, color representation, microscope type, objectives, optical filters, camera model and camera manufacturer. The static metadata may be essential for the selection of the feature extraction algorithm or model (e.g. trained artificial NN having an encoder-decoder architecture) since static metadata may determine the type of microscope image used by the feature extractor. Metadata can be dynamic which means metadata may be different for several microscope images being acquired. Accordingly, dynamic metadata may be specific for each images. Dynamic metadata can change over time and can be different per image. Dynamic metadata may relate to a temperature, a pH, carbon dioxide (CO₂) level, oxygen (O₂) level, radiation, electromagnetic waves (e.g. with respect to intensity, frequency, spectra, energy etc.) exposure time, gain or the like. Dynamic metadata may describe any parameter potentially having an influence on the state of the biological system due to (e.g. physical, mechanical, electrical, chemical, pharmacological, biological) interaction of, e.g. a surrounding or a substance, with the biological system.

The associated time is related to the microscope image of the biological system. The method 100 may be usable with more than one microscope images. For example, the method may be useable with a further microscope image at another associated time and corresponding further metadata. Further details regarding this aspect are described in conjunction with Fig. 2.

The microscope image may be generated by a microscope. Examples for microscopes are light microscopes, fluorescence microscopes, bright field microscopes, widefield microscopes, confocal microscopes, surgical microscopes, scanning electron microscopes or any type of microscope suitable to analyze the biological system with respect to its state. The type of microscope, and thus the specification of the microscope image, may be relevant for the algorithm or (e.g. trained artificial NN) model enabling feature extraction since the microscope image may serve as an input for the feature extraction model.

The future state is the state of the biological system at a time subsequent to the associated time. The feature state is the state of the biological system at a time in the future (at a time that has not happened so far). Accordingly, the method 100 predicts the state of the biological system in the future. An observer or investigator on the biological system can make use of the predicted future state for a time-series experiment, e.g. to change the workflow of the experiment, to manipulate or control the biological system intentionally based on set parameters or to change parameters to prolong the lifetime of the biological system or mitigate or stop degradation of the biological system.

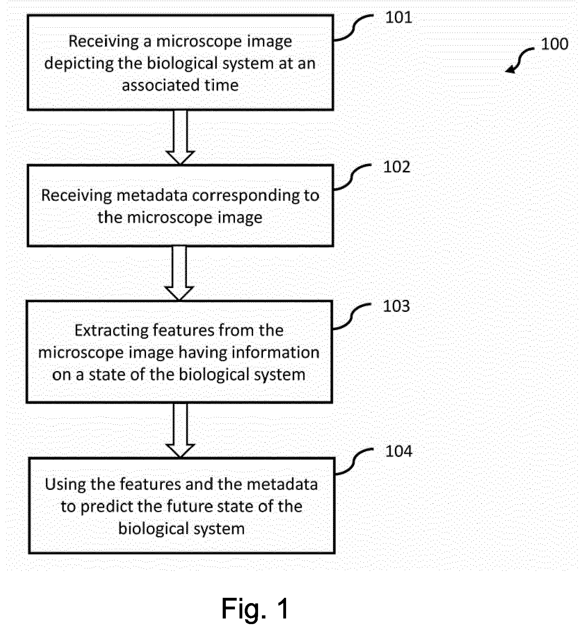

Although not explicitly illustrated in Fig. 1, the method 100 may comprise additional or optional aspects. For example, the method 100 may receive and use further microscope images and corresponding further metadata, may use a decoder of the trained artificial NN to reconstruct a segmented image, may identify a risk parameter of the metadata and/or may generate data for an external entity. Further details of the proposed method will be described in the following with reference to Figs. 2 to 11.

As described above, the method 100 can be used in conjunction with more than one microscope image and corresponding metadata. Since a further (e.g. second) microscope image and corresponding further (e.g. second) metadata are introduced hereafter, the microscope image and corresponding metadata may be considered as a first microscope image and corresponding first metadata. In the following, methods for predicting a feature state of the biological system are described in connection with models which can be an algorithm, software, or machine learning method to be implemented for feature extraction, image generation (e.g. segmented image generation by classification) or anomaly detection.

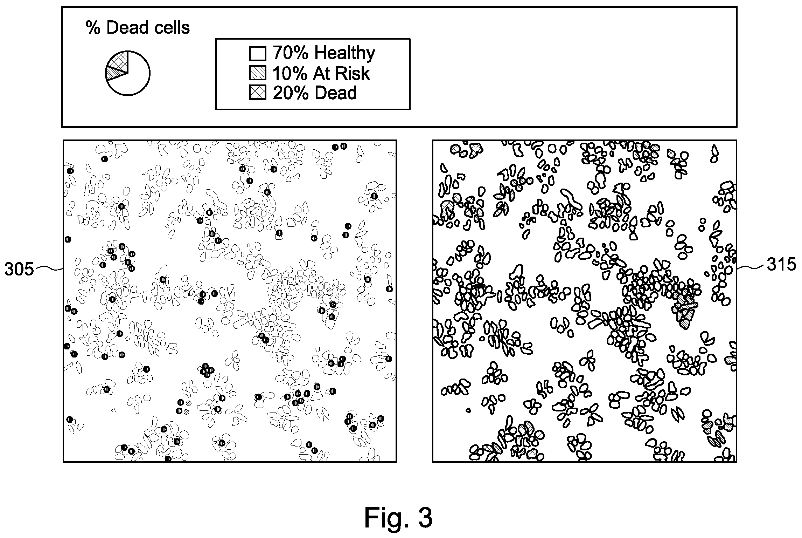

Fig. 2 shows another flowchart of an embodiment of a method 200 predicting a future state of a biological system. The implementation of the method 200 may be similar to the implementation of the method 100 described in connection with Fig. 1. The method 200 may comprises optional aspects indicated by the dashed lines. For better understanding, an overview of the aspects of the method 200 is given in connection with Fig 2. Some of these aspects are then described in conjunction with the Figs. 3-7 in further detail.

According to the method 200, (at least) a (first) microscope image 205a is received by a trained artificial NN 206 having an encoder-decoder architecture. The trained artificial NN 206 comprises an encoder (component, architecture) 207 and a decoder (component, architecture) 208. The encoder 207 receives the (at least first) microscope image 205a and extracts features of the microscope image 205a. In other words, extracting features from the microscope image (as described in connection with Fig. 1 in context with the step 103) comprises using the 207 encoder of the trained artificial NN 206 having the encoder-decoder architecture. The encoder 207 may generate a set of (extracted) features (e.g. a feature or state vector or matrix or tensor) comprising feature values (or any arbitrary symbol, code or type of representation for quantifying the state of the biological system).

The trained artificial NN 206 comprises the decoder 208 associated to the encoder 207. Generally, the decoder 208 can use an output of the encoder 207 (e.g. the feature vector) or any modified data being similar to the output of the encoder 207 (see description below). Based on the received input, the decoder 208 can generate a segmented image 213 depicting the biological system in segments according to classes. The trained artificial NN 206, comprising the encoder 207 and the decoder 208, can be trained by a set of microscope images as it is described in connection with Figs. 3-5.

The extracted features from the encoder 207 can be provided for an anomaly detection model 209. The anomaly detection model 209 can use the features from the encoder 207 and corresponding metadata 210 to predict the future state 211 of the biological system. Optionally, the output 211 of the anomaly detection model 209, comprising the predicted future state 211, can be modified (e.g. processed, aligned, trimmed, converted, transformed, split) such that a modified output 212 can be used by the decoder 208. Accordingly, the decoder 208 can reconstruct one or more segmented images 213 based on the future state being predicted. The segmented image 213 depicts the biological system as one or more segments according to the future state. With the segmented image 213, a visual representation of the future state 211 of the biological system can be provided for a user.

In sum, the proposed technique could be described by three models (or data processing units). The encoder 207 of the trained artificial NN 206 can be considered as a first model enabling feature extraction. The anomaly detection model 209 can be considered as a second model detecting anomalies based on the extracted features and the corresponding metadata to predict the future state 211. The decoder 208 of the trained artificial NN 206 can be an optional aspect and can be considered as a third model reconstructing the segmented image 213 for visual representation of the future state.

In the following, further details on the implementation of the method 200 are given with respect to cells only by way of example. It is to be understood that the proposed method can be used with any type of biological system as described above.

Fig. 3 illustrates an example of a microscope image 305 as an input for the trained artificial NN 206 and a corresponding output of the trained artificial NN 206 based on the microscope image 305. The microscope image 305 depicts a plurality of biological systems such as cells to be analyzed with respect to their states. The trained artificial NN 206 can be a classification network being trained for classifying biological systems according to their states. For example, the microscope image 305 depicts the plurality of biological systems at the associated time t. The trained artificial NN 206, having the encoder-decoder architecture, outputs a segmented image 315 based on the received microscope image 305. The segmented image 315 depicts each of the plurality of the biological systems according to a segment related to a state (e.g. healthy, dead or at risk for death) of the respective biological system. Accordingly, the segmented image 315 in Fig. 3 represents the current states of the plurality of biological systems at the associated time t.

It is to be noted, that the segmented image 315, giving information on the current states (rather than the predicted future state) of the plurality of the biological system, is discussed to describe the encoder-decoder architecture of the trained artificial NN 206. If the trained artificial NN receives features based on future states being predicted, the segmented image would depict the biological system according to the future states.

In the following, a possible (e.g. supervised) training of the artificial NN 206 is described.

Fig. 4 illustrates a flowchart for generating training data for the artificial NN 206 having the encoder-decoder architecture. A microscope 417 can generate (and optionally recoded) a plurality of microscope images for training the artificial NN 206 using a label-free modality. A human expert may label the microscope images (e.g. by manually annotating live and dead cells in the plurality of microscope images, for example using dots, polygons or bounding boxes and a class label) to provide labeled microscope image 419a based on his expertise and experience. Optionally, a labeled microscope images 419b can be generated by using a staining procedure such that correct labeling of the classes in the microscope images can be more reliable. By the staining procedure, uncertainty in detecting, e.g. live and dead cells (due to ambiguity or bias) can be reduced. The plurality of labeled microscope images 419a-b comprises labels for each biological system informing about its localization and class (e.g. live or dead) and serves as training data for the artificial NN 206. Accordingly, the artificial NN 206 can adjust its parameters (weights, biases in the encoder-decoder architecture) by supervised learning.

According to another example, the artificial NN 206 is trained without labeling. The artificial NN 206 may adapt its parameters only based on the microscope images by unsupervised learning (e.g. by recognizing patterns or clusters).

As indicated in Fig. 4, one may optionally record metadata 410 corresponding to the training data. If needed, the metadata 410 corresponding to the training data can be further used to train the anomaly detection model 209.

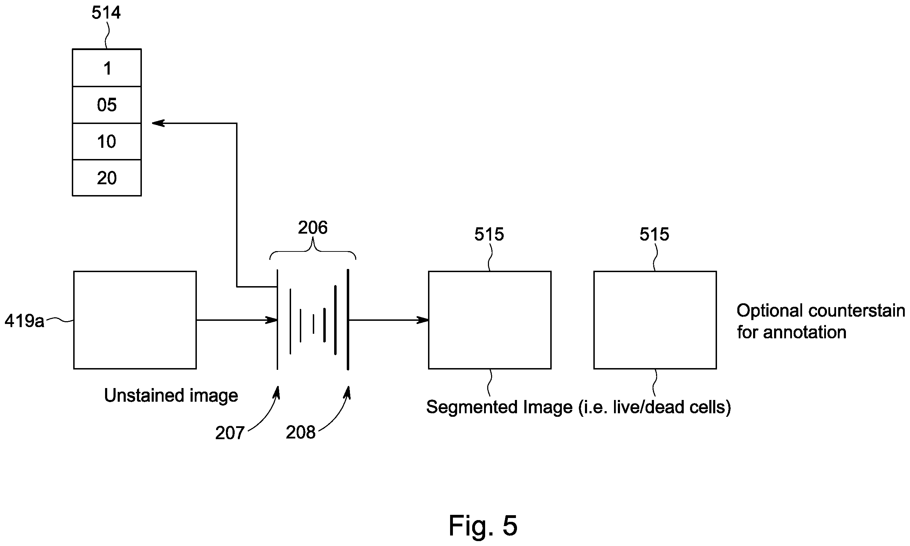

Fig. 5 illustrates a flowchart for exemplarily generating segmented images 515 by means of the artificial NN 206, having the encoder-decoder architecture, based on the training data being the labeled microscope images 419a-b. The flowchart in Fig. 5 may refer to the step for generating the segmented image 315 described in Fig. 3. According to Fig. 5, extracted features 514 from the encoder 207 can be directly used by the decoder 208 to evaluate the progress in training of the artificial NN 206 by assessing the segmented image 515 with respect to a target (e.g. an annotated microscope image). Optionally, a staining procedure can be used for assessing the output, and thus the training, of the trained artificial NN 206.

With the training data 419a-b used for training the artificial NN having the encoder-decoder architecture, metadata 410 and extracted features 514 can be optionally recorded to train the anomaly detection model 209 if needed.

As described in connection with Fig. 2, the anomaly detection model 209 uses the extracted features 214 from the encoder 207 of the trained artificial NN 206 and corresponding metadata 210 to predict the future state 211 of the biological system being observed.

For example, using the features 214 and the metadata 210 comprises detecting anomalies by means of a further trained artificial NN. The further trained artificial NN is trained based on a sequence of microscope images, depicting biological systems over time, and a corresponding sequence of metadata over the time. Accordingly, prediction of the future state 211 of the biological system can be enabled by a single microscope image 205a depicting the biological object at the associated time.

The further (or additional or second) trained artificial NN 209 may have learned to detect anomalies in the input data (features and corresponding metadata) due to its training based on the time-resolved training data. The training data is based on a set of microscope images and corresponding metadata related to biological systems (e.g. being of a same type compared to the biological system to be evaluated) at different time stamps. In other words, the further artificial NN 209 may have learned about a normal trend, development, pattern or correlation in the time-resolved training data. Hence, the further trained artificial NN 209 may have established a norm based on the training data. After training, the further trained artificial NN 209 can detect an anomaly (e.g. a deviation or outlier) in the input data with respect to the normal trend being established. If the further artificial NN 209 identifies that input data does not fit to the norm, an anomaly is detected.

As indicated by the dashed lines in Fig. 2, future state prediction can be achieved optionally by using (at least) a further microscope image 205b. Hence, the method 200 optionally further comprises receiving the further microscope image 205b depicting the biological system at another associated time (being different to the associated time t). Accordingly, the method 200 may further comprise receiving further metadata corresponding to the further microscope image 205b and extracting further features from the further microscope image 205b having information on the state of the biological system. The method 200 may comprise using the features and the further features and the metadata and the further metadata to predict the future state 211 by detecting anomalies based on a temporal development between the features and the further features and between the metadata 210 and further metadata. In other words, anomalies can be detected by time-resolved (or time-series) data provided by the microscope image 205a and at least one further microscope image 205b and the corresponding metadata 210 and the at least one further metadata. The anomaly detection model 209 can learn to identify what is normal based on e.g. the trend, development, pattern or correlation in the time-resolved data. For example, statistical methods based on thresholding, multivariate gaussian distribution or analysis of variance (ANOVA) can be used to identify an anomaly (e.g. significant deviation, outlier) with respect to the normal trend being observed within the time-resolved data.

For example, anomaly detection using on one or more microscope images and corresponding metadata can be enabled by baseline methods (e.g. naive/persistence, averaging forecasting), autoregressive and moving average (ARMA) methods or its variations such as auto regressive integrated moving average (ARIMA), autocorrelation functions (ACF) or reinforcement learning methods. Anomaly detection can be based on deep learning methods such as multilayer perceptrons (MPLs), convolutional neural networks (CNN) such as simple CNN models or multi-channel models and advanced multi-headed and multi-output models, long-short term memory (LSTM) architectures (e.g. simple LSTM models, stacked LSTMs, bidirectional LSTMs and encoder-decoder models for sequence-to-sequence learning), hybrids (e.g. hybrids of MLP, CNN and LSTM models such as CNN-LSTMs, ConvLSTMs) or deep transformer models for time series forecasting.

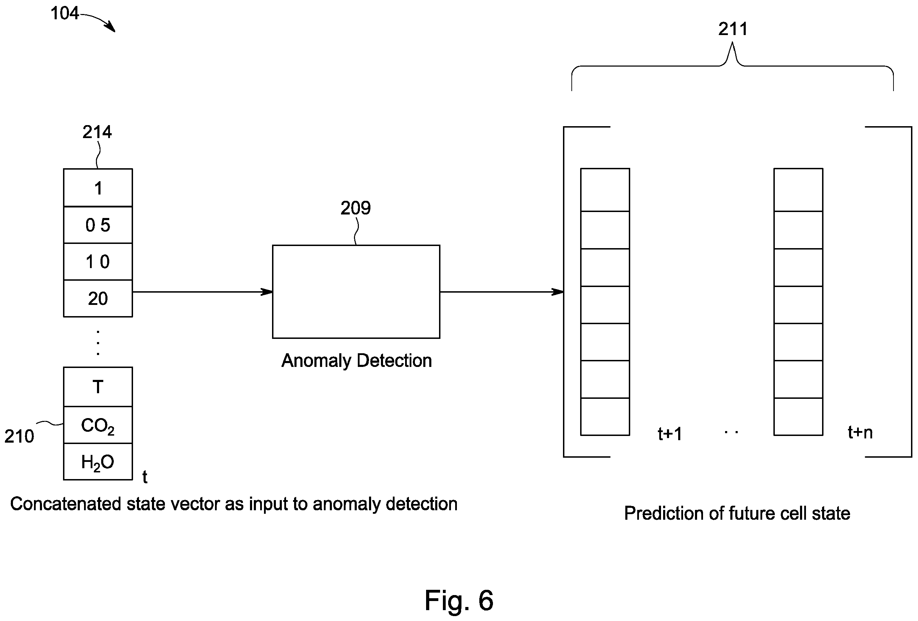

Fig. 6 illustrates a flowchart for the step 104 of using the extracted features 214 and the metadata 210 to predict the future state 211 of the biological system according to an example. The extracted features (e.g. vectors) 214 and the metadata 210 (at the associated time t) can be combined (or concatenated), e.g. by a (previous) intermediate step prior to anomaly detection or by the anomaly detection model 209 itself. As described above, the anomaly detection model 209 can detect outliers with respect to a norm being learned. Based on the techniques for anomaly detection, as exemplarily given above, a reliable forecast can be made for the future (e.g. cell) state of the biological system for the timesteps t+1, ..., t+n (being subsequent to the timestep t). The output of the anomaly detection model 209 can be similar to the combined input data with respect to dimensionality. It is noted that the anomaly detection techniques according to Fig. 6 can be also used in combination with two or more extracted feature vectors and corresponding metadata. The output of the anomaly detection model 209 can be aligned either by the anomaly detection model itself or a (subsequent) intermediate step. For example, the dimension of the output of the anomaly detection model 209 can be reduced by reversing the concatenation between the extracted features and the corresponding metadata. As a result, modified output 212 (see Fig. 2) can be further used to reconstruct the segmented image 213 being related to the future states 211 being predicted.

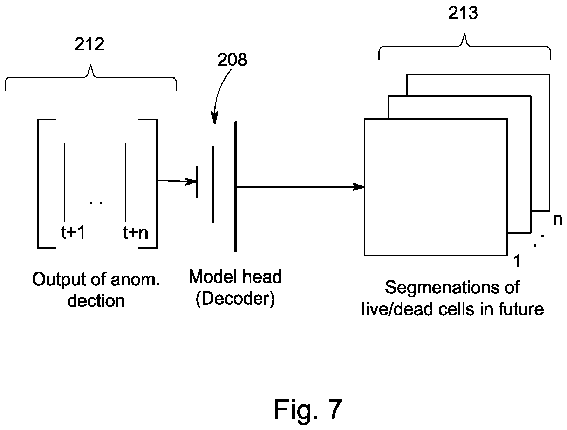

As indicated above in connection with Fig. 2, the method 200 can optionally further comprise using the decoder 208 of the trained artificial NN 206 having the encoder-decoder architecture to reconstruct the segmented image 213 based on the future state 211 being predicted. The segmented image 213 depicts the biological system as one or more segments according to the future state 211. Fig. 7 illustrates a flowchart for the step of reconstructing the segmented image 213 based on the future state 211 being predicted according to an example. The decoder 208 of the trained artificial NN 206, having the encoder-decoder architecture, can receive the modified output 212 of the anomaly detection model 209 as input data. Since the modified output 212 can be similar (with respect to type and dimensionality) to the extracted features 214 generated by the encoder 207, the segmented image 213 can be generated based on the predicted future state of the biological system. Accordingly, the segmented image 213 can be similar to the image 315 described in connection with Fig. 3, though with segments referring to the predicted future states (rather than the current states). Hence, the segmented image 213 can give an observer a visual impression on the future state 211 of the biological system. For example, if a plurality of cells is segmented regarding their future states 211, an observer can identify, e.g. how many cells are of a same future state (e.g. healthy, at risk, dead) or can determine clusters of cells having a same future state.

In view of the Figs. 2-7, the proposed method may be described as follows:
In total, the proposed method may be based on three neural networks: a first model may be a U-Net-like image-to-image model configured to create a segmentation of the microscope image. It may do semantic segmentation or instance segmentation with the classes corresponding to future states of the biological system (e.g. cells). The first model may be trained in a supervised learning regime using human annotated segmentation masks. The trained first model may be split, such that its encoder path can be used as a static feature extractor. The output of that feature extractor serves as latent vectors which are the input for the second model. The second model is an anomaly detection model which may receive as input the concatenation of the extracted features by the encoder of the first model to a state vector containing metadata (e.g. temperature T, pH, partial pressure pCO₂, partial pressure pO₂, H₂O level). For example, the result of the concatenation may be the input vector. The anomaly detection model may be a neural network by way of example. The output of the second model may be a plurality of vectors of the same dimension as the input vector. The plurality of vectors may represent consecutive future time steps predicted by the second model. There may be optionally a third model which can be produced during training of the first model. The third model may be equivalent to the decoding path of the first model. It may take as input a vector of the same dimensions as the extracted features by the first model. Only this time the features may have been predicted by the second model. The output of the second model may be truncated to the same number of features as are extracted by the first model. The output of the third model may be a segmented image. The prediction can be done for each time step predicted by the second model. So, the third model can reconstruct segmented cell images from latent space. The third model may give the user a visual representation of the predicted future state with respect to the biological system.

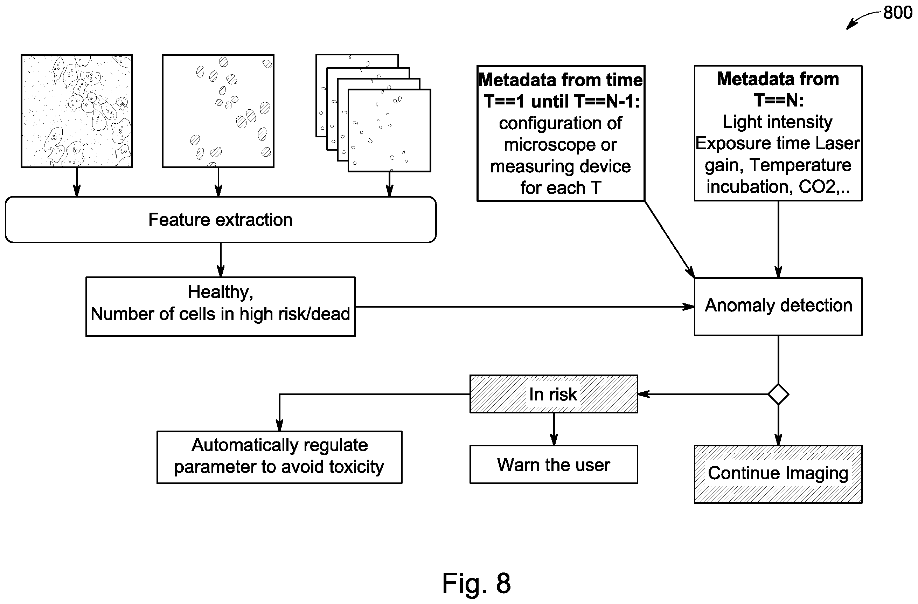

More details and aspects are mentioned in connection with the examples described above or below. The example shown in Fig. 2 (in connection with Figs. 3-7) may comprise one or more optional additional features corresponding to one or more aspects mentioned in connection with the proposed technique or one or more examples described above or below (e.g. Figs. 8-11).

According to some embodiments, the method for predicting the future state of the biological system further comprises identifying a risk parameter of the metadata. According to another example, the risk parameter may be derived from the metadata or may be dependent on the metadata. A risk parameter may be a parameter of the metadata or may be (e.g. linear) combination of them. A risk parameter has a positive correlation with a degradation of the state of the biological system with respect to the future state. For example, the risk parameter relates to an illumination property, a temperature, a humidity, an oxygen level, a carbon dioxide level or an agent having an influence on the state of the biological system. The risk parameter can be any parameter within the metadata. The risk parameter can be (partly or fully) responsible for (or correlated with) the degradation of the biological system. For example, a metric such as Euclidean distance, respect the values from a reference experiment, can be used to identify the risk parameter. If an anomaly is detected, the corresponding risk parameter may be identified if its value deviates from a mean or expected value beyond a threshold.

A manipulation of the risk parameter (e.g. reducing power, changing wavelength, increasing oxygen level, stabilizing temperature fluctuations) can potentially mitigate the degradation. For example, degradation of the biological system may occur if the future state of the biological system is different compared to the (current) state at the associated time. Degradation may occur, e.g. if the biological system deteriorates in a motion, breeding, activity, division, metabolism, reaction, sensitivity or the like. Degradation may occur if the biological system will die in case the biological system used to be alive at the associated time. Degradation may occur if there is a critical change in the state of the biological system.

According to some embodiments, the method for predicting the future state of the biological system further comprises generating data for an external entity having an influence on the risk parameter. The data comprises a command for the external entity to adapt a configuration related to the risk parameter for mitigating the degradation. For example, the external entity can be a microscope (used to acquire the microscope image of the biological system at the associated time), an incubator (accommodating the biological system), a thermostat, a gas regulator, a light source, a user of the microscope or the like. For example, the data can be a control signal to an external device or a visual or acoustic notification to the user. By generating the data, the external entity can be informed about the risk parameter likely being responsible for the degradation of the biological system. The external entity can automatically adjust the risk parameter to mitigate the degradation.

Fig. 8 illustrates another flowchart of an embodiment of the method 800 for predicting the future state of cells. The method 800 may be implemented similar to the implementation of the method 100, 200 described in connection with Figs. 1-2. During an experiment, microscope images can be obtained in the modality used by the trained artificial NN having the encoder-decoder architecture (e.g. transmitted light, or transmitted light with nuclei staining or transmitted light with any fluorescence markers). The trained artificial NN, having the encoder-decoder architecture may be a trained classifier. For example, the trained classifier may be based on ensemble learning to improve the performance of predictability.

If desired, at each time point of a time lapse experiment, the trained classifier may display a (current) risk evaluation for each individual cell, e.g. in a dashboard showing the percentage of healthy, dead or at risk, and with an overlay map displaying in colors each one of the categories (see Fig. 3). The trained classifier can extract features from the microscope images having information on the states of the cells. The extracted features may comprise information on the viability of the cells (e.g. level of health, number of cells in high risk for death, number of cells being dead,). The anomaly detection model can use the extracted features and the corresponding metadata (e.g. for each time T of the time lapse experiment) to predict the future state of the cells. During the experiment, if the cell culture displays a decline in healthy cells outside from a control experiment, (e.g. increased number of cells at risk and/or being dead), the parameters of the metadata (e.g. environmental, illumination conditions, etc.) can be evaluated and correlated with health. If there is a positive correlation, a user can be warned, or the microscope can automatically regulate configurations to minimize cell damage. Since it is possible that the conditions are constant and harming from the beginning of the experiment (e.g. illumination conditions generate phototoxicity in the long term), the microscope can regulate specific conditions if the risk of death increases (e.g. reduces exposure time).

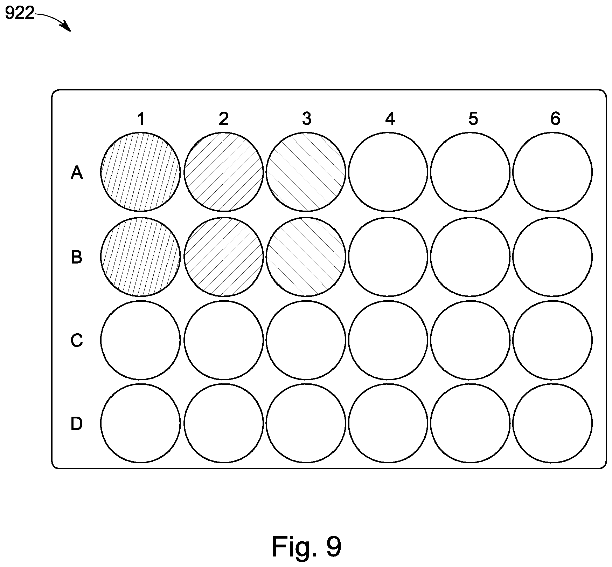

The proposed method can be also used to assess experimental conditions. Fig. 9 shows an example of a multiwell plate (a 24-well plate) 922 used in combination with the method for predicting the future state. The well plate 922 can be used as part of an experiment to set different conditions (e.g. exposure times) in different regions (different wells) and evaluate the optimal conditions of an experiment. For example, in a phototoxicity evaluation assay, using cells in a multiwell plate, each well (or well plate) could be set with a specific illumination condition. At the end of the experiment using a time lapse, it may be possible to assess which conditions are phototoxic and which are not, which could be then used as a control or reference. Hence, the proposed method can be used to optimize an assay and store the settings for longer or more expensive assays.

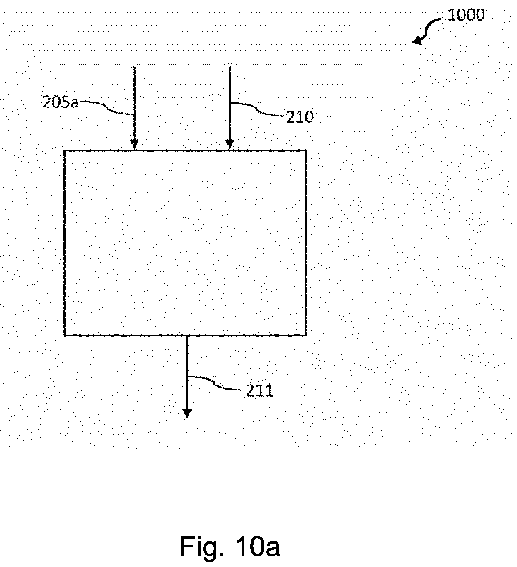

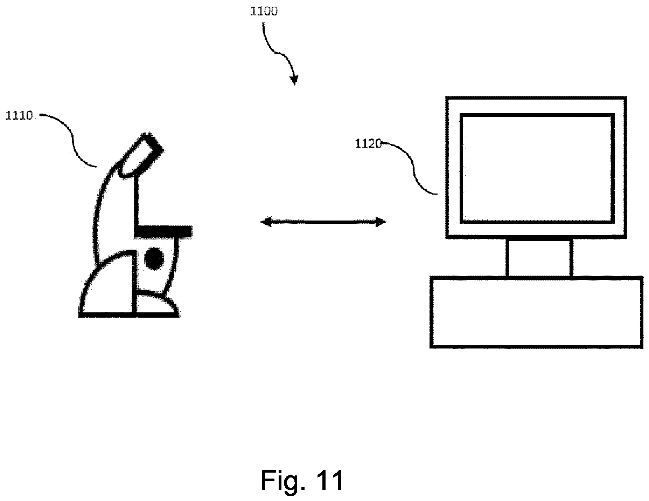

More details and aspects are mentioned in connection with the examples described above or below. The examples shown in Figs. 8 or 9 may comprise one or more optional additional features corresponding to one or more aspects mentioned in connection with the proposed technique or one or more examples described above or below (e.g. Figs. 10a-b, 11).

The method for predicting a future state of the biological system and one ore more aspects of the method described above in connection with the Figs. 1-9 can be implemented in an apparatus.

Fig. 10a shows an embodiment of an apparatus 1000 for predicting a future state of a biological system. The apparatus 1000 is configured to receive a microscope image 205a depicting a biological system at an associated time and receive metadata 210 corresponding to the microscope image. The apparatus 1000 is further configured to extract features from the microscope image having information on a state of the biological system and use the features and the metadata 210 to predict the future state 211 of the biological system. The apparatus 1000 may enable a reliable prediction of the future state 211 of the biological system by using one or more microscope images 205a of the biological system and corresponding metadata 210.

To this end, an apparatus with improved forecasting capabilities with respect to the future state of the biological system may be provided.

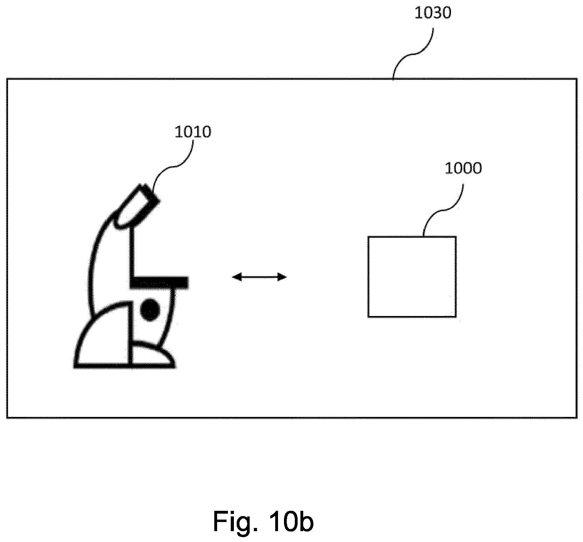

More details and aspects are mentioned in connection with the examples described above or below. The example shown in Fig. 10a may comprise one or more optional additional features corresponding to one or more aspects mentioned in connection with the proposed concept or one or more examples described above or below (e.g. Figs. 10b, 11).

The apparatus for predicting the future state of the biological system can be used in combination with a microscope.

Fig. 10b illustrates an embodiment of a system 1030 comprising a microscope 1010 configured to generate a microscope image depicting a biological system at an associated time. The system 1030 further comprises an apparatus 1000 for predicting a future state of a biological system as proposed. The apparatus 1000 is configured to receive the microscope image to predict a future state of the biological system. The microscope 1010 can directly supply the one or more microscope images and corresponding metadata to the apparatus for predicting the future state. Optionally, the apparatus 1000 can generate (feedback) data for the microscope 11010 to adapt its configuration related to the risk parameter for mitigating the degradation.

According to some embodiments, the proposed technique for predicting a future state may offer a workflow which might not need the use of staining steps for cell death and can address a larger sample for increased statistical confidence.

As stated, the workflow can be integrated in a microscope, so at each step of a time lapse, the cell culture may be evaluated, e.g. providing a risk score that indicates if cells are healthy or not. In case that cells are becoming progressively unhealthy, a model may infer which configuration parameters of the microscope are correlated with cell death. Experiments may be based on e.g. phototoxicity assessment, environmental assessment (e.g. related to an incubator) or simple analysis for cell death progression, e.g. in case of a drug experiment in a multi well plate. In case of a phototoxicity assessment, the microscope may receive feedback data to reduce the damaging effects of high energy illumination by reducing parameters such as exposure time or gain. For environmental assessment, the microscope or incubator can receive feedback data to prevent damaging by regulating a pointed parameter. Optionally, the user can use the health assessment to analyze the effect of a treatment such as specific phenotypes or drugs applied per wells or regions to detect when in the timelapse experiment the toxic or deadly effects are starting to be visible.

According to some embodiments, the proposed technique refers to an automated analysis of viability during experiments using microscopy. The method can offer a label-free cell assay with the possibility of identifying and adjusting what parameters affect cell viability in a cell assay. For that, a microscope can capture pictures in a label-free fashion (e.g. bright field, phase contrast, optionally together with fluorescence images). The microscope images can be classified by a NN trained to localize and classify live cells and dead cells. Further, a set of values (parameters coming from the configuration of the microscope hardware, like illumination, incubator) can be given to a model which may evaluate the current risk of toxicity. For example, if the phototoxicity risk increases, the microscope receives a feedback signal such that parameters for illumination conditions can be automatically tuned to reduce phototoxicity. Alternatively or optionally, a user can be warned about other relevant parameters directly correlated to cell death (e.g. temperature, O₂%, CO₂% values from incubator). The user may also be notified about the number of dead cells.

Some embodiments relate to a biological system being an organism formed by cells. The one or more individual cells can appear in the form of cell culture, organoid or a complete model organism (e.g. zebrafish or Caenorhabditis elegans worm). One can evaluate individual cells or the status of the overall system, e.g. by averaging, taking the median or adding up all the values of each individual cell. The individual values can be defined by a cell status from a cell (coming from an organism) which may be in a certain state. The status is the precision of describing a situation (e.g. in medical/biological terms) while state is a general description and can be expressed in a mathematical way using a quantity or a vector of quantities. For example, in a live/death assay, the status can be the probability of a cell to be in an apoptotic/cell death process, where 0% would be "not at all" and 70% could indicate that the cell starts to show some signs. At 80% the cell might definitely go into cell death or apoptosis and with 90% or higher the cell might be dead. In a cell cycle analysis, the state indicates in which part of the cell cycle is each cell. E.g. Cell 1 is in "G1" and Cell 2 is in "SI". The proposed method may define states in a simplified way, which can be the outcome of a simple classification. For example, the state can be defined by a vector.

In machine learning terminology there is something called the latent space, which is the result of a creating a machine learning model (like a deep learning network) to some data (like images/text/signals). When specific data is given, this data falls in specific regions of the latent space. By obtaining the values of the latent space, one can get a vector (or a tensor), of reduced dimensionality that can be used by a classifier to make decisions (i.e. to classify the object).

In some embodiments, extracted features and metadata are concatenated. The metadata and the extracted features may be grouped in a multivariate signal, where time series analysis can be performed if images are recorded over time. By using the multivariate signal, forecasting can be enabled by anomaly detection. An anomaly happens when a signal changes with respect to a standard or usual signal. For example, one may determine outliers (elements of the signal that go far away from a range defined by statistical parameters, e.g. mean and standard deviation), or trends, which are unexpected increments or decrements of a certain value from one variable over a period time. Anomalies (e.g. trends) can be also detected by doing forecasting. If a model for forecasting is applied, it may be possible to prevent further damages to a working system (e.g. a small leak in the incubation chamber of O₂ can be detected as a trend where cell status degrades and O₂ values starts to decline).

As described above, the proposed method and apparatus for predicting a future state of a biological object is usable in combination with a microscope.

Some embodiments relate to a microscope comprising an apparatus for predicting a future state of a biological system as described in connection with one or more of the Figs. 1 to 10 (in context with the corresponding method). Alternatively, a microscope may be part of or connected to a system as described above. Fig. 11 shows a schematic illustration of a system 1100 configured to perform a method described herein. The system 1100 comprises a microscope 1110 and a computer system 1120. The microscope 1110 is configured to take microscope images and is connected to the computer system 1120. The computer system 1120 is configured to execute at least a part of a method described herein. The computer system 1120 may be configured to execute a machine learning algorithm. The computer system 1120 and microscope 1110 may be separate entities but can also be integrated together in one common housing. The computer system 1120 may be part of a central processing system of the microscope 1110 and/or the computer system 1120 may be part of a subcomponent of the microscope 1110, such as a sensor, an actor, a camera or an illumination unit, etc. of the microscope 1110.

The computer system 1120 may be a local computer device (e.g. personal computer, laptop, tablet computer or mobile phone) with one or more processors and one or more storage devices or may be a distributed computer system (e.g. a cloud computing system with one or more processors and one or more storage devices distributed at various locations, for example, at a local client and/or one or more remote server farms and/or data centers). The computer system 1120 may comprise any circuit or combination of circuits. In one embodiment, the computer system 1120 may include one or more processors which can be of any type. As used herein, processor may mean any type of computational circuit, such as but not limited to a microprocessor, a microcontroller, a complex instruction set computing (CISC) microprocessor, a reduced instruction set computing (RISC) microprocessor, a very long instruction word (VLIW) microprocessor, a graphics processor, a digital signal processor (DSP), multiple core processor, a field programmable gate array (FPGA), for example, of a microscope or a microscope component (e.g. camera) or any other type of processor or processing circuit. Other types of circuits that may be included in the computer system 1120 may be a custom circuit, an application-specific integrated circuit (ASIC), or the like, such as, for example, one or more circuits (such as a communication circuit) for use in wireless devices like mobile telephones, tablet computers, laptop computers, two-way radios, and similar electronic systems. The computer system 1120 may include one or more storage devices, which may include one or more memory elements suitable to the particular application, such as a main memory in the form of random access memory (RAM), one or more hard drives and/or SSDs, and/or one or more drives that handle removable media such as compact disks (CD), flash memory cards, digital video disk (DVD), and the like. The computer system 1120 may also include a display device, one or more speakers, and a keyboard and/or controller, which can include a mouse, trackball, touch screen, voice-recognition device, or any other device that permits a system user to input information into and receive information from the computer system 1120.

Some or all of the method steps may be executed by (or using) a hardware apparatus, like for example, a processor, a microprocessor, a programmable computer or an electronic circuit. In some embodiments, some one or more of the most important method steps may be executed by such an apparatus.

Depending on certain implementation requirements, embodiments of the invention can be implemented in hardware or in software. The implementation can be performed using a non-transitory storage medium such as a digital storage medium, for example an HDD, an SSD, a floppy disc, a DVD, a Blu-Ray, a CD, a ROM, a PROM, and EPROM, an EEPROM or a FLASH memory, having electronically readable control signals stored thereon, which cooperate (or are capable of cooperating) with a programmable computer system such that the respective method is performed. Therefore, the digital storage medium may be computer readable.

Some embodiments according to the invention comprise a data carrier having electronically readable control signals, which are capable of cooperating with a programmable computer system, such that one of the methods described herein is performed.

Generally, embodiments of the present invention can be implemented as a computer program product with a program code, the program code being operative for performing one of the methods when the computer program product runs on a computer. The program code may, for example, be stored on a machine-readable carrier.

Other embodiments comprise the computer program for performing one of the methods described herein, stored on a machine-readable carrier.

In other words, an embodiment of the present invention is, therefore, a computer program having a program code for performing one of the methods described herein, when the computer program runs on a computer.

A further embodiment of the present invention is, therefore, a storage medium (or a data carrier, or a computer-readable medium) comprising, stored thereon, the computer program for performing one of the methods described herein when it is performed by a processor. The data carrier, the digital storage medium or the recorded medium are typically tangible and/or non-transitionary. A further embodiment of the present invention is an apparatus as described herein comprising a processor and the storage medium.

A further embodiment of the invention is, therefore, a data stream or a sequence of signals representing the computer program for performing one of the methods described herein. The data stream or the sequence of signals may, for example, be configured to be transferred via a data communication connection, for example, via the internet.

A further embodiment comprises a processing means, for example, a computer or a programmable logic device, configured to, or adapted to, perform one of the methods described herein.

A further embodiment comprises a computer having installed thereon the computer program for performing one of the methods described herein.

A further embodiment according to the invention comprises an apparatus or a system configured to transfer (for example, electronically or optically) a computer program for performing one of the methods described herein to a receiver. The receiver may, for example, be a computer, a mobile device, a memory device or the like. The apparatus or system may, for example, comprise a file server for transferring the computer program to the receiver.

In some embodiments, a programmable logic device (for example, a field programmable gate array) may be used to perform some or all of the functionalities of the methods described herein. In some embodiments, a field programmable gate array may cooperate with a microprocessor in order to perform one of the methods described herein. Generally, the methods are preferably performed by any hardware apparatus.

Embodiments may be based on using a machine-learning model or machine-learning algorithm. Machine learning may refer to algorithms and statistical models that computer systems may use to perform a specific task without using explicit instructions, instead relying on models and inference. For example, in machine-learning, instead of a rule-based transformation of data, a transformation of data may be used, that is inferred from an analysis of historical and/or training data. For example, the content of images may be analyzed using a machine-learning model or using a machine-learning algorithm. In order for the machine-learning model to analyze the content of an image, the machine-learning model may be trained using training images as input and training content information as output. By training the machine-learning model with a large number of training images and/or training sequences (e.g. words or sentences) and associated training content information (e.g. labels or annotations), the machine-learning model "learns" to recognize the content of the images, so the content of images that are not included in the training data can be recognized using the machine-learning model. The same principle may be used for other kinds of sensor data as well: By training a machine-learning model using training sensor data and a desired output, the machine-learning model "learns" a transformation between the sensor data and the output, which can be used to provide an output based on non-training sensor data provided to the machine-learning model. The provided data (e.g. sensor data, meta data and/or image data) may be preprocessed to obtain a feature vector, which is used as input to the machine-learning model.

Machine-learning models may be trained using training input data. The examples specified above use a training method called "supervised learning". In supervised learning, the machine-learning model is trained using a plurality of training samples, wherein each sample may comprise a plurality of input data values, and a plurality of desired output values, i.e. each training sample is associated with a desired output value. By specifying both training samples and desired output values, the machine-learning model "learns" which output value to provide based on an input sample that is similar to the samples provided during the training. Apart from supervised learning, semi-supervised learning may be used. In semi-supervised learning, some of the training samples lack a corresponding desired output value. Supervised learning may be based on a supervised learning algorithm (e.g. a classification algorithm, a regression algorithm or a similarity learning algorithm). Classification algorithms may be used when the outputs are restricted to a limited set of values (categorical variables), i.e. the input is classified to one of the limited set of values. Regression algorithms may be used when the outputs may have any numerical value (within a range). Similarity learning algorithms may be similar to both classification and regression algorithms but are based on learning from examples using a similarity function that measures how similar or related two objects are. Apart from supervised or semi-supervised learning, unsupervised learning may be used to train the machine-learning model. In unsupervised learning, (only) input data might be supplied and an unsupervised learning algorithm may be used to find structure in the input data (e.g. by grouping or clustering the input data, finding commonalities in the data). Clustering is the assignment of input data comprising a plurality of input values into subsets (clusters) so that input values within the same cluster are similar according to one or more (pre-defined) similarity criteria, while being dissimilar to input values that are included in other clusters.

Reinforcement learning is a third group of machine-learning algorithms. In other words, reinforcement learning may be used to train the machine-learning model. In reinforcement learning, one or more software actors (called "software agents") are trained to take actions in an environment. Based on the taken actions, a reward is calculated. Reinforcement learning is based on training the one or more software agents to choose the actions such, that the cumulative reward is increased, leading to software agents that become better at the task they are given (as evidenced by increasing rewards).

Furthermore, some techniques may be applied to some of the machine-learning algorithms. For example, feature learning may be used. In other words, the machine-learning model may at least partially be trained using feature learning, and/or the machine-learning algorithm may comprise a feature learning component. Feature learning algorithms, which may be called representation learning algorithms, may preserve the information in their input but also transform it in a way that makes it useful, often as a pre-processing step before performing classification or predictions. Feature learning may be based on principal components analysis or cluster analysis, for example.

In some examples, anomaly detection (i.e. outlier detection) may be used, which is aimed at providing an identification of input values that raise suspicions by differing significantly from the maj ority of input or training data. In other words, the machine-learning model may at least partially be trained using anomaly detection, and/or the machine-learning algorithm may comprise an anomaly detection component.

In some examples, the machine-learning algorithm may use a decision tree as a predictive model. In other words, the machine-learning model may be based on a decision tree. In a decision tree, observations about an item (e.g. a set of input values) may be represented by the branches of the decision tree, and an output value corresponding to the item may be represented by the leaves of the decision tree. Decision trees may support both discrete values and continuous values as output values. If discrete values are used, the decision tree may be denoted a classification tree, if continuous values are used, the decision tree may be denoted a regression tree.

Association rules are a further technique that may be used in machine-learning algorithms. In other words, the machine-learning model may be based on one or more association rules. Association rules are created by identifying relationships between variables in large amounts of data. The machine-learning algorithm may identify and/or utilize one or more relational rules that represent the knowledge that is derived from the data. The rules may e.g. be used to store, manipulate or apply the knowledge.

Machine-learning algorithms are usually based on a machine-learning model. In other words, the term "machine-learning algorithm" may denote a set of instructions that may be used to create, train or use a machine-learning model. The term "machine-learning model" may denote a data structure and/or set of rules that represents the learned knowledge (e.g. based on the training performed by the machine-learning algorithm). In embodiments, the usage of a machine-learning algorithm may imply the usage of an underlying machine-learning model (or of a plurality of underlying machine-learning models). The usage of a machine-learning model may imply that the machine-learning model and/or the data structure/set of rules that is the machine-learning model is trained by a machine-learning algorithm.

For example, the machine-learning model may be an artificial neural network (ANN). ANNs are systems that are inspired by biological neural networks, such as can be found in a retina or a brain. ANNs comprise a plurality of interconnected nodes and a plurality of connections, so-called edges, between the nodes. There are usually three types of nodes, input nodes that receiving input values, hidden nodes that are (only) connected to other nodes, and output nodes that provide output values. Each node may represent an artificial neuron. Each edge may transmit information, from one node to another. The output of a node may be defined as a (non-linear) function of its inputs (e.g. of the sum of its inputs). The inputs of a node may be used in the function based on a "weight" of the edge or of the node that provides the input. The weight of nodes and/or of edges may be adjusted in the learning process. In other words, the training of an artificial neural network may comprise adjusting the weights of the nodes and/or edges of the artificial neural network, i.e. to achieve a desired output for a given input.

Alternatively, the machine-learning model may be a support vector machine, a random forest model or a gradient boosting model. Support vector machines (i.e. support vector networks) are supervised learning models with associated learning algorithms that may be used to analyze data (e.g. in classification or regression analysis). Support vector machines may be trained by providing an input with a plurality of training input values that belong to one of two categories. The support vector machine may be trained to assign a new input value to one of the two categories. Alternatively, the machine-learning model may be a Bayesian network, which is a probabilistic directed acyclic graphical model. A Bayesian network may represent a set of random variables and their conditional dependencies using a directed acyclic graph. Alternatively, the machine-learning model may be based on a genetic algorithm, which is a search algorithm and heuristic technique that mimics the process of natural selection.

As used herein the term "and/or" includes any and all combinations of one or more of the associated listed items and may be abbreviated as "/".

Although some aspects have been described in the context of an apparatus, it is clear that these aspects also represent a description of the corresponding method, where a block or device corresponds to a method step or a feature of a method step. Analogously, aspects described in the context of a method step also represent a description of a corresponding block or item or feature of a corresponding apparatus.

### List of reference signs

- 100, 200, 800: Method for predicting a future state of a biological system
- 101: Receiving a microscope image depicting the biological system at an associated time
- 102: Receiving metadata corresponding to the microscope image
- 103: Extracting features from the microscope image having information on a state of the biological system
- 104: Using the features and the metadata to predict the future state of the biological system
- 205a-b,: Microscope image
- 305, 419a-b:
- 206: Trained artificial NN having an encoder-decoder architecture
- 207: Encoder of trained artificial NN having an encoder-decoder architecture
- 208: Decoder of trained artificial NN having an encoder-decoder architecture
- 209: Further trained artificial NN
- 210,410: Metadata
- 211: Future state
- 212: Modified output
- 213, 315,: Segmented image
- 515:
- 214, 514: Extracted features
- 417, 1010: Microscope
- 1110:
- 922: Well plate
- 1000: Apparatus for predicting a future state of a biological system
- 1030: System
- 1100: Microscope system
- 1120: Computer system

## Claims

1. A method (100, 200, 800) for predicting a future state of a biological system, comprising:
receiving (101) a microscope image depicting the biological system at an associated time;
receiving (102) metadata corresponding to the microscope image;
extracting (103) features from the microscope image having information on a state of the biological system; and
using (104) the features and the metadata to predict the future state of the biological system.

2. The method (100) according to claim 1, wherein
the state of the biological system is related to at least one of a health, an activity, and a growth of the biological system.

3. The method (100) according to claim 1 or 2, wherein
the metadata (210) comprises information on at least one of a configuration of a microscope, used for generating the microscope image, a surrounding, an agent interacting with the biological system at the associated time, a temperature, a pH, a partial pressure of carbon dioxide, a partial pressure of oxygen, a humidity, a culture condition of the biological system, a type or amount of a buffer solution, nutrient, antibiotic or growth factor of the biological system at the associated time.

4. The method (100) according to one of the claims 1 to 3, wherein
extracting (103) features from the microscope image (205a) comprises using an encoder (207) of a trained artificial neural network (206) having an encoder-decoder architecture.

5. The method (100) according to one of the claims 1 to 4, wherein
using (104) the features and the metadata comprises detecting anomalies by means of a further trained artificial neural network (209), the further trained artificial neural network (209) being trained based on a sequence of microscope images, depicting biological systems over time, and a corresponding sequence of metadata over the time.

6. The method (100) according to one of the claims 1 to 5, further comprising:
receiving a further microscope image (205b) depicting the biological system at another associated time;
receiving further metadata corresponding to the further microscope image;
extracting further features from the further microscope image having information on the state of the biological system; and
using the features and the further features and the metadata and the further metadata to predict the future state (211) by detecting anomalies based on a temporal development between the features and the further features and between the metadata and further metadata.

7. The method (100) according to claim 4, further comprising:
using a decoder (208) of the trained artificial neural network (206) having the encoder-decoder architecture to reconstruct a segmented image (213) based on the future state (211) being predicted, the segmented image (213) depicting the biological system as one or more segments according to the future state (211).

8. The method (100) according to one of the preceding claims, further comprising:
identifying a risk parameter of the metadata (210), the risk parameter having a positive correlation with a degradation of the state of the biological system with respect to the future state.

9. The method (100) according to claim 8, further comprising:
generating data for an external entity having an influence on the risk parameter, the data comprises a command for the external entity to adapt a configuration related to the risk parameter for mitigating the degradation.

10. The method (100) according to claim 8 or 9, wherein
the risk parameter relates to an illumination property, a temperature, a humidity, an oxygen level, a carbon dioxide level or an agent having an influence on the state of the biological system.

11. An apparatus (1000) for predicting a future state (211) of a biological system, configured to
receive a microscope image (205a) depicting a biological system at an associated time; receive metadata (210) corresponding to the microscope image (205a);
extract features (214) from the microscope image (205a) having information on a state of the biological system; and
use the features (214) and the metadata (210) to predict the future state (211) of the biological system.

12. A system (1030), comprising:
a microscope (1010) configured to generate a microscope image (205a) depicting a biological system at an associated time; and
an apparatus (1000) for predicting a future state (211) of a biological system according to claim 11, the apparatus (1000) configured to receive the microscope image (205a) to predict a future state (211) of the biological system.

13. A computer program with a program code for performing the method (100) according to one of claims 1 to 10 when the computer program is executed by a processor.
